# FASCICULE DE BREVET EUROPEEN

(11) **EP 0 877 807 B1**
(45) Date de publication et mention de la délivrance du brevet: **25.06.2003**
(21) Numéro de dépôt: 97901145.9
(22) Date de dépôt: 22.01.1997
(51) Int. Cl.: C12N 15/18, C07K 14/61, A61K 39/385, A23K 1/165

(54) **MOLECULES BIOLOGIQUEMENT ACTIVES PLUS PARTICULIEREMENT PEPTIDIQUES AYANT UN EFFET POTENTIALISATEUR DE L'ACTIVITE BIOLOGIQUE DE L'HORMONE DE CROISSANCE**
BIOLOGISCH AKTIVE STOFFE INSBESONDERE PEPTIDE, DIE EINEN VERSTÄRKENDEN EFFEKT AUF DIE BIOLOGISCHE AKTIVITÄT DES WACHSTUMSHORMONS HABEN
BIOLOGICALLY ACTIVE AND PARTICULARLY PEPTIDE MOLECULES HAVING A POTENTIATING EFFECT ON GROWTH HORMONE BIOLOGICAL ACTIVITY

(30) Priorité: 26.01.1996 FR 9600956
(43) Date de publication de la demande: 18.11.1998
(73) Titulaire: Carelli, Claude Marcel Henri, 92150 Suresnes (FR)
(72) Inventeur: PORTETELLE, Daniel, B-5881 Meux-la-Bruyère (BE); RENAVILLE, Robert, B-5030 Gembloux (BE)
(74) Mandataire: Desaix, Anne
(86) Numéro de dépôt international: FR9700127
(87) Numéro de publication internationale: WO97027298

(56) Documents cités:
- EP-A- 0 303 488
- WO-A-94/05697
- MOLECULAR IMMUNOLOGY, vol. 28, no. 1/2, Janvier 1991 - Février 1991, pages 41-50, XP000608941 R. ASTON ET AL.: "Antigenic Structure of Bovine Growth Hormone: Location of a Growth Enhancing Region"
- J. PROTEIN CHEM. (1992), 11(6), 657-63 CODEN: JPCHD2;ISSN: 0277-8033, 1992, XP000609083 LEHRMAN, S. R. ET AL: "Identification and characterization of an anti-isoaspartic acid monoclonal antibody"
- BIOCHEMISTRY (1987), 26(24), 7774-8 CODEN: BICHAW;ISSN: 0006-2960, 1987, XP002018134 BREMS, D. N. ET AL: "Helical formation in isolated fragments of bovine growth hormone"

## Description

La présente invention concerne une molécule biologique active de type peptidique qui, lorsqu'elle est liée de façon covalente à certains peptides transporteurs et/ou certains adjuvants est capable d'induire in vivo un effet potentialisateur de l'activité biologique de l'hormone de croissance GH.

La potentialisation de l'activité biologique de la GH par des anticorps, plus particulièrement de l'activité somatogénique, fut d'abord observée par Holder et al. (1980), qui basèrent leurs études sur la mesure d'une incorporation de sulfates radioactifs dans le cartilage costal, ainsi que sur le gain de poids induit par la GH chez les souris naines de type Snell.

Trois études basées sur les mêmes paramètres effectuées par Holder et al (1985) et Aston et al (1986 et 1987), montrèrent que la majorité des anticorps étudiés avait un effet potentialisateur de l'action biologique de la GH. Les travaux de Wallis et al (1987) ont confirmé le phénomène d'augmentation de l'activité somatogénique de l'hormone de croissance bovine par des anticorps anti-GH. Ceux-ci basèrent leurs études sur la mesure du taux plasmatique IGF-I (Insuline growth factor) et du gain de poids de rats hypophysectomisés de race Wistar.

En particulier, la demande de brevet européen EP 284 406 (COOPERS ANIMAL HEALTH LTD), décrit un peptide ayant une homologie de structure primaire avec une séquence continue de résidus aminoacide de l'hormone de croissance dans la région s'échelonnant entre les positions 35 à 53 ou de peptides à réactivité croisée, ledit peptide pouvant être utilisé dans une composition antigénique afin de potentialiser les effets de l'hormone de croissance chez un vertébré. Les expériences décrites par Aston et al correspondent à une administration in vivo d'anticorps, soit sous forme de sérums, lorsque ceux-ci sont obtenus chez le mouton, soit sous forme d'anticorps monoclonaux, et cette administration passive d'anticorps dirigés contre le peptide ou dirigés contre l'hormone naturelle GH native, complexés à ladite hormone, augmente l'activité biologique de celle-ci. Il n'a pas été démontré que le peptide 35-53 de l'hormone de croissance pouvait être utilisé dans le cadre d'une immunisation active.

Des récents travaux réalisés avec des anticorps potentialisateurs, sembleraient indiquer que si la GH seule se fixe aux hépatocytes, le complexe GH-MAb ou le complexe formé de l'hormone selon l'invention et d'un anticorps potentialisateur monoclonal, se fixerait préférentiellement au niveau des cellules sinusoïdales (cellules de Kupffer) (Tans et al (1994)). Ainsi, on pourrait dès lors admettre que, la synthèse d'IGP-1 et d'lGF BP3 au niveau des hépatocytes (Massart et al (1993)) pourrait être également augmentée par l'action desdits complexes au niveau de ces cellules sinusoïdales.

Il est décrit dans la demande de brevet EP 137 234 (THE WELLCOME FOUNDATION LIMITED), que certains anticorps de l'hormone de croissance sont capables de potentialiser l'activité de celle-ci, alors qu'on sait que, généralement, de tels anticorps, au moins in vivo, avaient tendance à antagoniser son action. De tels anticorps peuvent en outre, être produits in situ par la « vaccination » de l'animal hôte avec un fragment spécifique de l'hormone de croissance, de sorte qu'une classe d'anticorps polyclonaux de spécificité restreinte soit créée, ce qui pourrait potentialiser l'activité de l'hormone endogène.

Dans la présente invention, il est montré que certaines séquences de l'hormone de croissance associées à une molécule porteuse et/ou un adjuvant sont capables de potentialiser l'activité de ladite hormone chez un vertébré. La découverte du fragment peptidique de la présente invention est le résultat d'une recherche basée dans un premier temps sur la sélection d'anticorps monoclonaux (MAb) de la classe des IgG dont les réactivités vis-à-vis de l'hormone de croissance, en particulier de l'hormone de croissance bovine, paraissaient les plus élevées. Après purification desdits MAb, quatre types ont été retenus. L'effet de ces derniers sur la réponse en IGF-I plasmatique induite par une injection unique de bGH chez le rat hypophysectomisé immature a été testé par Massart et al (1993). On a pu enfin identifier un épitope reconnu par un anticorps potentialisateur dénommé 2H4, cet anticorps étant capable de reconnaître spécifiquement une séquence peptidique située dans l'hélice α N°3 de l'hormone de croissance native.

Toute référence de structure primaire, secondaire ou tertiaire de la GH se réfère à celle décrite dans Scanes C.G. et al (1995).

Le but de la présente invention est de fournir un moyen permettant la potentialisation de l'hormone de croissance GH de façon efficace et plus durable que les moyens de l'art antérieur. Notamment, la présente invention fournit un peptide, ou haptène, capable lorsqu'il est lié de façon covalente à un peptide transporteur et/ou un adjuvant, d'induire in vivo, un effet potentialisateur de l'activité biologique de cette hormone.

Un tel effet peut être le résultat d'une immunisation active de type vaccination, où l'administration de l'haptène induit notamment la production d'anticorps spécifiques in vivo.

La présente invention concerne donc une construction peptidique ayant un effet potentialisateur de l'activité biologique de l'hormone de croissance GH, caractérisée en ce qu'elle consiste en l'une des structures suivantes :
a) au moins un haptène constitué de tout ou partie des séquences allant de la position 104 à 113 de ladite hormone de croissance, lié de façon covalente à un peptide transporteur,
b) la structure en a) associée de façon covalente ou non à un adjuvant,
c) au moins un haptène constitué de tout ou partie des séquences allant de la position 104 à 113 de ladite hormone de croissance, associé de façon covalente ou non à un adjuvant.

L'identification précise de cette séquence de la GH ou « peptide GH », est issue de l'analyse d'un certain nombre d'anticorps monoclonaux anti-GH, responsables d'un effet potentialisateur lors de l'administration du complexe anticorps-GH. Ce fragment peptidique, dont la séquence peptidique issue de GH peut être en outre considérée comme une molécule du type haptène, est reconnu par l'anticorps 2H4. D'autre part, parmi les anticorps ayant les meilleurs effets potentialisateurs, on peut noter que l'affinité de 2H4 pour GH est faible par rapport à celles d'autres anticorps.

Selon une construction peptidique préférée de l'invention, ladite séquence de l'hormone de croissance GH est comprise dans la séquence suivante :
GTSDRVYEKL

De préférence encore, ladite séquence de l'hormone GH est sélectionnée parmi les séquences suivantes :
TSDRVYEKL
GTSDRVYEK
SDRVYEKL
TSDRVYEK
GTSDRVYE

Ces cinq peptides correspondent respectivement aux régions 105-113, 104-112, 106-113, 105-112, 104-111, de l'hormone de croissance GH telle que publiée dans Scanes et al (1995) et peuvent être obtenus par des techniques classiques de synthèse peptidique.

Un haptène n'étant par définition pas immunogène, le peptide de l'invention est couplé de façon covalente à un transporteur. A titre d'exemple, de tels transporteurs peuvent être de façon avantageuse des séquences peptidiques issues de l'ovalbumine, la KLH (Keyhole Limpet Hemocianine) ou l'albumine et plus particulièrement les peptides suivants : 323-339 de l'ovalbumine,
378-398 ou 379-398 ou 378-397 ou 378-396 ou 378-395 du peptide CS.T3, décrit par Sinigaglia et al,
45-60 de la protéine 1A du virus syncitial respiratoire, 120-140 de la protéine enveloppant l'ARN génomique du virus de l'hépatite B.

Ces séquences sont couplées de façon covalente à l'extrémité carboxylique ou aminée du « peptide GH ».

Le fragment peptidique de l'invention peut être lié de façon covalente à, outre les molécules transporteuses ci-dessus, un adjuvant du type N-acetyl-muramyl (MAP) associé à un peptide, comme le MDP ou ses dérivés.

Selon un mode de réalisation préféré du fragment peptidique selon l'invention, l'adjuvant est le muramyl-dipeptide (MDP) ou un de ses dérivés tel que le MDP-lysine Lys(NH₂)-D-isoGlu-L-Ala-NMc-Nur.

Le dérivé de MDP est alors couplé de façon covalente à l'extrémité carboxylique du peptide constitué du « peptide GH » et du transporteur, d'une façon sensiblement similaire à ce qui est décrit dans EP 89290.

Le fragment peptidique selon l'invention peut en outre être lié à une séquence connue comme, ou présumée T-dépendante avec ou sans MDP, ou ses dérivés, ou alternativement lié ou englobé dans des liposomes. Il peut être ainsi administré dans des milieux aqueux ou huileux, par voie orale, sous forme d'injection sous-cutanée, intra-musculaire ou transmucosale, par « pellets » (polymères biodégradables contenant le produit) et par implantation de systèmes de relargage par micropompes. D'autres adjuvants, tels que le PAO, seul ou associé à la lécithine (EP 445710), Zn (OH)₂ ou HBW 538 (DRUGS.EXP.CLIN.RES.17(9) 1991 445-450) associé à Al (OH)₃ peuvent être associés, de façon covalente ou non, au peptide GH transporteur.
Enfin, bien entendu, sur une même molécule transporteuse, plusieurs fragments peptidiques selon l'invention peuvent être fixés, sans pour autant que l'effet potentialisateur soit affecté.

Les « peptides GH », ou peptides GH couplés au peptides transporteurs peuvent être synthétisés par voie chimique, par des méthodes connues de l'homme de métier, comme par exemple la méthode de Merrifield.

Une telle approche qui propose une immunisation active est une technique présentant de nombreux avantages en comparaison de l'administration d'anticorps. En effet, une immunisation active permet, à l'instar de la vaccination, un effet pérenne, alors que l'administration d'anticorps n'a qu'un effet transitoire ; en outre la production de peptides est sensiblement moins onéreuse que celle des anticorps ou de celle de la GH native.

Entre également dans le cadre de la présente invention un acide nucléique recombinant incorporant une séquence nucléotidique codant un haptène constitué de tout ou partie des séquences allant de la position 104 à la position 113 de l'hormone de croissance GH, et le cas échéant, un peptide transporteur, ledit acide nucléique recombinant étant approprié pour la production des constructions peptidiques selon l'invention, dans des cellules procaryotes ou eucaryotes. Un tel acide nucléique recombinant peut être utilisé :
- soit pour transfecter des cellules procaryotes ou eucaryotes et leur faire produire *in vitro* lesdits peptides ;
- soit comme principe actif d'un médicament potentialisateur de la GH ; les peptides sont alors produits *in vivo* par un mécanisme similaire à celui décrit dans WANG B. et al (1993).

La présente invention vise également une composition immunogène comprenant un fragment peptidique, ou un acide nucléique recombinant, décrits ci-dessus.

Dans une telle composition, un adjuvant peut être ajouté, qu'il soit couplé au peptide comme décrit ci-dessus ou non. Une composition selon l'invention peut avantageusement être utilisée comme additif alimentaire pour stimuler la croissance et/ou la lactation des animaux, notamment les bovins, ovins, porcins et autres vertébrés (poissons, marsupiaux, humains...). Elle peut être destinée à une administration orale, par exemple sous forme d'additif alimentaire, ou à une administration rectale, sous-cutanée, intramusculaire ou transmucosale, « pellets » et systèmes de micropompes ; en particulier des liposomes peuvent être utilisés à titre de vecteur dans une composition incorporant le peptide GH, couplé ou non avec un transporteur et à un adjuvant.

Pour un effet promoteur de la croissance ou de la lactation chez les animaux, la composition est formulée de telle façon que le peptide de l'invention soit administré à raison de 0,01 µg à 10 µg par kg.
Les peptides et les compositions de l'invention peuvent également être utilisés chez l'homme dans la fabrication d'un médicament contre les troubles de la croissance, que ceux-ci résultent d'un déficit en hormone de croissance endogène ou d'une pertubation du métabolisme de cette dernière. Les formulations de ces compositions seraient également telles que ci-dessus définies.
Sans être limitatifs, les exemples et les figures ci-dessous montrent l'effet particulièrement avantageux des constructions peptidiques de l'invention, et notamment en comparaison avec d'autres promoteurs de croissance.

### Description détaillée

### 1) Détermination de la séquence du « peptide GH »

Dans une étape préliminaire, il était nécessaire de sélectionner parmi les différents anticorps monoclonaux dirigés contre la bGH, ceux qui présentaient une certaine affinité pour celle-ci. Ceux-ci paraissait être en effet ceux qui serait les plus susceptibles d'interférer de façon positive ou négative avec l'activité biologique de l'hormone.

Pour l'obtention de ces anticorps, on a utilisé une technique rapide d'immunisation permettant d'obtenir des anticorps monoclonaux spécifiques décrite par Holmdahl et al, (1985) et Mirza et al, (1987). La bGH a été utilisée comme antigène.

Les anticorps obtenus ont été caractérisés en ce qui concerne :
- L'isotypie
- La réactivité en RIA avec la bGH marqué à l'iode 125
- La réactivité en ELISA (titration additivité, capture)
- les réactivités en WESTERN-BLOT.

Les anticorps ont ensuite été purifiés à partir des ascites par la technique décrite dans Bruck et al (1982), et une fois la purification terminée, les solutions d'anticorps obtenues ont fait l'objet d'une mini-électrophorèse sur gel dénaturant au SDS (PHAST SYSTEM). Cette opération a pour but de contrôler l'isolement des immunoglobulines.

Il a été déterminé par une technique rapide la capacité des MAb purifiés à former des concentrations importantes de complexes immuns avec la bGH en solution, par une méthode notamment décrite par Massart. Cette réactivité est mesurée par la capacité de l'anticorps à former un complexe avec la bGH marquée à l'I¹²⁵ dans la condition de précipitation en présence de PEG (polyéthylène glycol).
Ceci a permis de sélectionner un anticorps présentant la réactivité souhaitée et appelée 2H4 qui a été étudié quant à son affinité avec la bGH. Les résultats des caractéristiques physico-chimiques de cet anticorps sont exposés dans la thèse de S. Massart et le tableau I ci-dessous.

**Tableau 1**

| **N° clone** | **Statut anticorps** | **Isotype** | **RIA** | | | **React Titr.** | **ELISA Capt.** | **React. W.B.** |
|---|---|---|---|---|---|---|---|---|
| | | | **(1)** | **(2)** | **(3)** | | | |
| 2H4 | S,A | IgG1 (A) | +++ | +++ | ++++ | 10,3 | 9,3 | ++ |
| S : surnageant de culture | | | | | | | | |
| A : liquide d'acsite. | | | | | | | | |
| (A) : Isotype qui est déterminé à partir de liquide d'ascite dilué et non de surnageant de culture | | | | | | | | |
| ++ : précipitation moyenne | | | | | | | | |
| +++ : précipitation forte | | | | | | | | |
| ++++ : précipitation très forte. | | | | | | | | |

React Titr. : Titration des MAb anti-bGH des liquides d'ascite (représente la valeur de DO observée s'étalant de 0,200 (1) à 2.000 (10))
ELISA Capt. ; capteur de bGH biotinée à l'aide des anticorps adsorbés sur plastique (représente le nombre de dilutions d'anticorps montrant la DO maximale observée.

### b) Cartographie de l'épjtope reconnu par l'anticorps 2H4 :

Les travaux décrits ci-dessus ont donc permis de déterminer l'épitope du MAb 2H4 par des études de cartographie épitopiques effectuées notamment selon Beattie et Holder, (1994).

Une batterie de peptides de 8 acides aminés a été synthétisée et mise en réaction avec les anticorps monoclonaux. Les peptides qui lient les anticorps ont été identifiés par spectrométrie. Le poids moléculaire des complexes anticorps-peptides est nettement plus élevé que celui des peptides seuls.

Il a aussi été démontré que le site de fixation à l'anticorps était le peptide 104-113 de la structure de la GH.

Cette région 104-113 se situe au niveau de l'hélice α N°3 de la GH, région très peu étudiée de l'hormone GH, en raison du fait que les peptides de cette hélice présentent un faible activité de radiorécepteurs (Scanes et al, Ed. 1995). On sait toutefois que les peptides de cette région présentent une activité promotrice de croissance significative, l'hélice α3 est en outre la région du second site de liaison de la protéine de liaison GH (GHBP). En effet, pour que la GH se lie à son récepteur hépatique, il est nécessaire qu'elle constitue un dimère avec deux GH BP afin d'induire l'effet promoteur de croissance selon le modèle de Fuh , (1992) (Science, 256, 1677-1678). En l'absence de cette dimérisation de la GH ou GHBP, aucun antagonisme d'action ne se produit et aucun effet promoteur de croissance n'est alors obtenu. On peut dès lors supposer que l'anticorps potentialisateur facilite ou modifie cette liaison de GHPP au niveau du site de liaison, ce qui pourrait expliquer l'effet avantageux de ce « peptide GH ».

### Exemples

Des expériences in vivo réalisées dans le cadre de la présente invention ont été menées sur 19 lots, à raison 5 rats par lot, de rats femelles hypophysectomisées de la race Wistar. Ces animaux ont été hypophysectomisés à l'âge de 4 semaines et gardés ensuite en observation durant une semaine. Durant une semaine d'observation supplémentaire, on procède à des pesées et les animaux ayant gagné plus de 5 grammes au bout de sept semaines sont éliminés. On considère en effet, que l'hypophysectomie de ces animaux est incomplète. Ils ne sont donc pas aptes à être utilisés dans le cadre des présents travaux.

Chaque animal sélectionné, reçoit un traitement substitutif d'hormone thyroidienne T4 et de cortisone. Ces hormones sont administrées quotidiennement par voie sous-cutanée à raison de 50 µg /100 g de poids corporel pour la cortisone et de 1 µg /100 g de poids corporel pour la T4. On observe généralement un léger gain de poids des animaux suite à l'injection de ces hormones. Ce traitement de complémentation hormonale est appliqué durant une semaine, pendant laquelle on procède toujours aux pesées quotidiennes. Ces animaux sont dès lors aptes à être utilisés pour une évaluation comparative de l'activité somatogénique de la bGH incubée ou non avec différentes constructions peptidiques ayant pour peptide GH la séquence 104-113 ou la séquence 35-53, décrite dans EP 284406.

Au cours de cette évaluation qui a été effectuée durant 50 jours, on a cherché à mesurer, par le gain de poids et le taux d'IGF-I, l'augmentation de l'activité hormonale de la GH porcine administrée sous forme de complexe avec des anticorps dirigés contre le peptide 104-113 comparé au peptide 35-53 de la GH.

Le peptide 104-113 a été rendu immunogène par couplage à différentes molécules porteuses d'origine peptidique pour former les constructions peptidiques ci-après décrites et qui ont ensuite été injectées aux individus, couplées ou non à des adjuvants d'origines diverses, notamment des peptides dérivés du MDP, des huiles minérales, des produits d'origine bactérienne ou divers hydroxydes. Cette injection a été effectuée sous forme d'une composition comprenant lesdites constructions peptidiques à raison de 0,01 µg à 10 µg de composition par kg (masse du rat).

Parallèlement, des rats témoins également hypophysectomisés reçoivent des injections de GH porcine à raison de 0,01 µg à 10 µg par kg, soit en présence d'immunoglobulines normales (témoins négatifs), soit en présence d'anticorps dirigés contre peptide 35-53 de la GH (témoins supposés positifs).

Les deux immunogènes (35-53-OVA et 35-53-SRIF) ont été décrits dans la demande de brevet européen EP284406 comme efficaces pour l'obtention d'anticorps capables d'augmenter l'activité de la GH chez des rats Wistar hypophysectomisés. Dans les présentes expériences, le peptide 35-53 de la GH a en outre été rendu immunogène par le même type de couplages covalent que pour le peptide 104-113.

Les immunogènes 104-113-OVA, 104-113-LysMDP, 104-113-SRIF, 104-113-SRIF-LysMDP ont donc été comparés à des immunogènes ayant les mêmes types de conjugués (molécules transporteuses ou adjuvants) mais dont le peptide 104-113 a été remplacé par le peptide 35 -53.

Les méthodes utilisées pour la conjuguaison de l'OVA et de SRIF peuvent être celles décrites dans la demande de brevet EP284406. En outre, le couplage au LysMDP peut être effectué selon la méthode décrite par Carelli et al. dans le brevet européen EP 89290.

La détection des anticorps anti-peptides et anti-GH a également été effectuée selon la méthode décrite dans la demande de brevet européen EP284406.

L'injection sous-cutanée a été appliquée aux jours J-1, J-15 et J-40 et des saignées ont été effectuées aux jours J-1, J-7, J-21, J-45, et une saignée finale à J-50.

### Descriptif des lots :

| | |
|---|---|
| Lot 1 | PBS (témoin négatif). |
| Lot 2 | ACF (adjuvant complet de Freund, 2eme témoin négatif) |
| Lot 3 | AIF (adjuvant incomplet de Freund, 3eme témoin négatif) |
| Lot 4 | PBS + 104-113 conjugué à l'OVA |
| Lot 5 | ACF + 104-113 conjugué à l'OVA |
| Lot 6 | PBS + 35-53 conjugué à l'OVA |
| Lot 7 | ACF + 35-53 conjugué à l'OVA |
| Lot 8 | PBS + 104-113 conjugué au SRIF |
| Lot 9 | ACF + 104-113 conjugué au SRIF |
| Lot 10 | PBS + 35-53 conjugué au SRIF |
| Lot 11 | ACF + 35-53 conjugué au SRIF |
| Lot 12 | PBS + 104-113 conjugué à MDPLys |
| Lot 13 | PBS + 35-53 conjugué à MDPLys |
| Lot 14 | PBS + 104-113 conjugué au SRIF conjugué à MDPlys |
| Lot 15 | PBS + 35-53 conjugué au SRIF conjugué à MDPLys |
| Lot 16 | AIF + 104-113 conjugué à MDPLys |
| Lot 17 | AIF + 35-53 conjugué à MDPLys |
| Lot 18 | AIF + 104-113 conjugué au SRIF conjugué à MDPLys |
| Lot 19 MDPLys | AIF + 35-53 conjugué au SRIF conjugué à |

Ont alors été comparés sur les différents lots :
- l'induction d'anticorps potentialisateurs selon la méthode décrite dans Massart (1989) pages 27 et 34.
- l'induction d'anticorps anti-peptide, ainsi que le taux d'anticorps anti-peptide reconnaissant le GH native
- l'évolution pondérale
- le taux d'IGF-I plasmatique

En fin de traitement, après pesage des individus dans l'ensemble des lot, les animaux sont sacrifiés par décapitation sans anesthésie pour la détermination du taux d'IGF-I, et l'évolution pondérale et les réponses en IGF-I des différents groupes expérimentaux ont été comparées sur les différents lots.

Pour la détermination du taux d'IGF-I, le sang du tronc est recueilli dans des tubes en verre. Après rétraction du caillot (2 heures à 4°C) les échantillons sont centrifugés et les sera sont conservés à -20°C.

On procède ensuite à l'extraction de I'IGF-1 selon la méthode décrite par Renaville et al (1993) page 444. Le taux de récupération de l'IGF-1 par cette technique est de 93 % ; plus de 99 % des protéines de liaison sont éliminées. L'IGF-I ainsi extrait est alors dosé par radioimmunoessai en utilisant l'antisérum Rab 2 (Renaville et al (1993)).

On peut tout d'abord remarquer la similitude qui existe entre les deux types de résultats, puisqu'il existe effectivement une corrélation importante entre les gains pondéraux et les réponses en IGF-I.

En effet, à la dose de 100 µg /rat, la bGH induit des taux d'IGF moyens de 60 ng/ml. Cette valeur est huit fois plus élevée que les taux de base chez les animaux hypophysectomisés contrôlés.

Les résultats de cette expérience montrent que les constructions peptidiques avec le peptide GH induisent une augmentation de l'activité de l'hormone. Cette augmentation est reflétée par la concentration d'IGF-I obtenue qui est supérieure à celle des animaux injectés par la bGH seule.

Les résultats de cette expérience montrent également qu'une corrélation existe entre le taux d'anticorps circulants reconnaissant l'hormone native (GH) et la prise de poids des rats immunisés. En particulier, les lots recevant les peptides soit conjugués à l'OVA et injectés en présence de ACF ainsi que ceux couplés directement au MDPLys et injectés dans le PBS se détachent nettement des autres lots. L'OVA est donc un meilleur transporteur que le SRIF, même quand ce dernier est couplé à son tour au MDPLys.

Le couplage direct des peptides au MDPLys donne de bons résultats quand il est administré en PBS plutôt qu'en AIF. Il doit être également noté que dans les lots recevant le peptide 35-53, le lot 7 donne un résultat supérieur aux lots 13 et 17 mais cependant moins performant que les lots 5 et 12.

### BIBLIOGRAPHIE

(1) Aston et al. « Potention of the somatogenic and lactogenic activity of human GH with Mab » (1986) J. Endocrinol.110 381-388.
(2) Aston et al. « Enhancement of the of bovine GH activity with Mab » (1987) Mol. Immunol. 24; 143-150.
(3) Beattie et al. Mol. Endocrinol. (1994) 8; 1103-1110.
(4) Bruck et al. (1982) « The Step purification of mouse Mab from ascitic fluid by DEAE affigel blue chromatography » J. of 53, Immunological Methodology 313-316.
(5) Fuh et al. Science (1994), 256 ,1677-1678.
(6) Holder et al. « Effects of GH, prolactin and thyroxine on body weight, somatomedin-like activity and in vivo sulphation of cartilage in hypopituitary Snell dwarf mice » (1980) J. Endocrinol. 85; 34'-47.
(7) Holder et al. «Monoclonal antibody-mediated Enhancement of GH activity in vivo» (1980) J. Endocrinol. 107; R9-12.
(8) Holmdahl et al. «A rapid and efficient immunisation protocol for production of monoclonal antibodies reactive with autoantigens » (1985) J. Immunol. Meth. 83; 379-384.
(9) Maiter et al. (1989) Endocrinology, 124 , 2604-2611.
(10) Massart.S Thèse de Doctorat « Etude de l'influence in vivo de Mab anti-bGH sur l'activité somatogénique de l'hormone b-GH» (1989) Faculté des Sciences Agronomiques de Gembloux -Belgique.
(11). Massart et al. (1993) « Mab to bGH potentiate hormonal activity in vivo by enhancing GH binding to hepatic somatogenic receptors » J. Endocrinology 139,383-393.
(12) Mirza et al. « A comparison of spleen and lymph node cells as fusion partners of the raising of Mab after different routes of immunisation » (1987) J. Immunol. Meth. 105; 235-243.
(13) Renaville et al (1993) « Changes in the hypophysiai gonadal axis during the onset of puberty in young bulls » J. of Reproduction and Fertility 99, 443-449.
(14) Scanes et al. « Growth Hormone Chemistry » (1995) Growth Hormone, Ed. Harvey S., ScanesC.G. & Daugahaday W.H., CRC Press, London, pp 1-25.
(15) Sinigaglia et al. (1988) Nature; vol 336, 778-780.
(16) Tans et al (1994) « Uptake by rat liver of bGH free or bound to a Mab », Bio Cell 82, 45-49.
(17) Wallis et al (1987). « Mab to bGH potentiate effects of the hormone on somatomedin-C levels and growth of hypophysectomised rats » Boichem. Biophys. Res. Comm.. 149; 187-193.
(18) Wang et al. (1993) PNAS 90 4156-4160.

## Revendications

1. Construction peptidique ayant un effet potentialisateur de l'activité biologique de l'hormone de croissance GH, **caractérisée en ce qu'**elle consiste en l'une des structures suivantes :
a) au moins un haptène constitué de tout ou partie des séquences allant de la position 104 à 113 de ladite hormone de croissance, lié de façon covalente à un peptide transporteur,
b) la structure en a) associée de façon covalente ou non à un adjuvant,
c) au moins un haptène constitué de tout ou partie des séquences allant de la position 104 à 113 de ladite hormone de croissance, associé de façon covalente ou non à un adjuvant.

2. Construction peptidique selon la revendication 1 caractérisée en que la séquence de l'haptène est sélectionnée parmi les séquences suivantes :
TSDRVYEKL
GTSDRVYEKL
SDRVYEKL
TSDRVYEK
GTSDRVYE

3. Construction peptidique selon l'une quelconque des revendications 1 à 2 **caractérisée en ce que** le peptide transporteur est sélectionné parmi les peptides ayant les séquences suivantes :
323-339 de l'ovalbumine,
378-398 ou 379-398 ou 378-397 ou 378-396 ou 378-395 du peptide CS.T3,
45-60 de la protéine 1A du virus syncitial respiratoire,
120-140 de la protéine enveloppant l'ARN génomique du virus de l'hépatite B.

4. Construction peptidique selon l'une quelconque des revendications 1 à 3, **caractérisée en ce que** l'adjuvant est le muramyl-dipeptide (MDP) ou un de ses dérivés tel que le MDP-Lysine, le cas échéant lié de façon covalente à l'extrémité carboxylique dudit haptène.

5. Acide nucléique recombinant incorporant une séquence nucléotidique codant un haptène constitué de tout ou partie des séquences allant de la position 104 à la position 113 de l'hormone de croissance GH, et le cas échéant, un peptide transporteur, ledit acide nucléique recombinant étant approprié pour la production des constructions peptidiques selon l'une quelconque des revendications 1 à 3, dans des cellules procaryotes ou eucaryotes.

6. Composition comprenant une construction peptidique selon l'une quelconque des revendications 1 à 4.

7. Composition selon la revendication 6, **caractérisé en ce qu'**elle comprend une construction peptidique selon l'une quelconque des revendications 1 à 4 et un adjuvant.

8. Utilisation d'une composition selon l'une quelconque des revendications 6 ou 7 dans la fabrication d'additifs alimentaires.

9. Utilisation selon la revendication 8 **caractérisée en ce que** la composition est formulée dans des liposomes.

10. Utilisation de compositions selon l'une quelconque des revendications 6 ou 7 dans la préparation d'additifs alimentaires permettant de stimuler la croissance et la lactation des vertébrés qui l'ingèrent

11. Utilisation d'acide nucléique recombinant selon la revendication 5 pour la fabrication d'une composition destinée à l'immunisation active de vertébrés.

## Claims

1. A peptide construction having a potentiating effect on the biological activity of growth hormone GH, **characterized in that** it consists of one of the following structures :
a) at least one hapten consisting of all or part of the sequence extending from position 104 to position 113 of said growth hormone, said hapten being linked covalently to a transporter peptide,
b) the structure of a), covalently or not covalently associated to an adjuvant,
c) at least one hapten consisting of all or part of the sequence extending from position 104 to position 113 of said growth hormone, said hapten being covalently or not covalently associated to an adjuvant.

2. Peptide construction according to Claim 1, **characterized in that** its amino acid sequence is selected from the following sequences:
TSDRVYEKL
GTSDRVYEK
SDRVYBKL
TSDRVYEK
GTSDRVYE

3. Peptide construction according to either of Claims 1 and 2, **characterized in that** the transporter peptide is selected from the Peptides having the following sequences:
323-339 of ovalbumin
376-398 or 379-398 or 378-397 or 378-396 or 378-395 of the peptide CS.T3
45-60 of the A1 protein of the respiratory syncitial virus
120-140 of the protein enveloping the genomic RNA of the hepatitis B virus.

4. Peptide construction according to any one of Claims 1 to 3, **characterized in that** the adjuvant is the muramyl-dipeptide (MDP) or one of its derivatives such as MDP-lysine, optionally linked covalently to the carboxyl terminus of said hapten.

5. A recombinant nucleic acid incorporating a nucleotide sequence coding for a hapten consisting of all or part of the sequence extending from position 104 to position 113 of said growth hormone, and optionally, a transporter peptide, said recombinant nucleic acid being appropriate for the production of peptide constructions according to any one of Claims 1 to 3, in prokaryotic or eukaryotic cells.

6. Composition containing a peptide construction according to any one of Claims 1 to 4.

7. A composition according to Claim 6, **characterized in that** it comprises a peptide construction according to any one of Claims 1 to 4 and an adjuvant.

8. Use of composition according to Claim 6 or 7 in the manufacture of feedstuffs additives.

9. Use according to Claim 8, **characterized in that** the composition is formulated in liposomes.

10. Use according to Claims 6 or 7, in the preparation of feedstuffs additives permitting to stimulate the growth and lactation of the vertebrates which ingest them.

11. Use of recombinant nucleic acid according to Claim 5 for the manufacture of a composition designed for the active immunization of vertebrates.

## Patentansprüche

1. Peptidkonstruktion, die einen verstärkenden Effekt auf die biologische Aktivität des Wachstumshormons GH hat, **dadurch gekennzeichnet, dass** sie aus einer der folgenden Strukturen besteht:
a) mindestens einem Hapten, bestehend aus den gesamten oder einem Teil der Sequenzen, die sich von Position 104 bis 113 des Wachstumshormons erstrecken, kovalent an ein Trägerpeptid gebunden,
b) der Struktur aus a), kovalent oder nicht kovalent mit einem Adjuvans assoziiert,
c) mindestens einem Hapten, bestehend aus den gesamten oder einem Teil der Sequenzen, die sich von Position 104 bis 113 des Wachstumshormons erstrecken, kovalent oder nicht kovalent mit einem Adjuvans assoziiert.

2. Peptidkonstruktion nach Anspruch 1, **dadurch gekennzeichnet, dass** die Haptensequenz ausgewählt ist aus den folgenden Sequenzen:
TSDRVYEKL
GTSDRVYEKL
SDRVYEKL
TSDRVYEK
GTSDRVYE.

3. Peptidkonstruktion nach einem der Ansprüche 1 bis 2, **dadurch gekennzeichnet, dass** das Trägerpeptid ausgewählt ist aus den Peptiden mit den folgenden Sequenzen:
323-339 von Ovalbumin,
378-398 oder 379-398 oder 378-397 oder 378-396 oder 378-395 von Peptid CS.T3,
45-60 von Protein 1A des Respiratory-Syncytial-Virus,
120-140 des die genomische DNA des Hepatitis-B-Virus umhüllenden Proteins.

4. Peptidkonstruktion nach einem der Ansprüche 1 bis 3, **dadurch gekennzeichnet, dass** das Adjuvans Muramyldipeptid (MDP) oder eines seiner Derivate, wie das MDP-Lysin, ist, gegebenenfalls kovalent gebunden an das Carboxylende des Haptens.

5. Rekombinante Nucleinsäure, umfassend eine Nucleotidsequenz, die ein Hapten codiert, das aus den gesamten oder einem Teil der Sequenzen besteht, die sich von Position 104 bis Position 113 des Wachstumshormons GH erstrecken, und gegebenenfalls ein Trägerpeptid, wobei die rekombinante Nucleinsäure für die Produktion von Peptidkonstruktionen nach einem der Ansprüchen 1 bis 3 in prokaryontischen oder eukaryontischen Zellen geeignet ist.

6. Zusammensetzung, enthaltend eine Peptidkonstruktion nach einem der Ansprüche 1 bis 4.

7. Zusammensetzung nach Anspruch 6, **dadurch gekennzeichnet, dass** sie eine Peptidkonstruktion nach einem der Ansprüche 1 bis 4 und ein Adjuvans enthält.

8. Verwendung einer Zusammensetzung nach einem der Ansprüche 6 oder 7 bei der Herstellung von Lebensmittelzusätzen.

9. Verwendung nach Anspruch 8, **dadurch gekennzeichnet, dass** die Zusammensetzung in Liposomen formuliert ist.

10. Verwendung von Zusammensetzungen nach einem der Ansprüche 6 oder 7 bei der Herstellung von Lebensmittelzusätzen, mit denen das Wachstum und die Milcherzeugung der Wirbeltiere, die sie zu sich nehmen, angeregt werden kann.

11. Verwendung der rekombinanten Nucleinsäure nach Anspruch 5 für die Herstellung einer Zusammensetzung zur aktiven Immunisierung von Wirbeltieren.
